# EUROPEAN PATENT SPECIFICATION

(11) **EP 3 055 001 B1**
(45) Date of publication and mention of the grant of the patent: **12.12.2018**
(21) Application number: 14852757.5
(22) Date of filing: 07.10.2014
(51) Int. Cl.: A61M 5/00, A61M 25/02, A61M 5/32, A61M 5/42

(54) **VEIN ACCESS NEEDLE GUIDE ASSEMBLY AND METHODS OF USE**
FÜHRUNGSANORDNUNG FÜR EINE VENENZUGANGSNADEL UND VERFAHREN ZUR VERWENDUNG
ENSEMBLE DE GUIDE D'AIGUILLE D'ACCÈS VEINEUX ET PROCÉDÉS D'UTILISATION

(30) Priority: 07.10.2013 US 201361887839 P
(43) Date of publication of application: 17.08.2016
(73) Proprietor: V-Align, Inc., Dobbs Ferry, NY 10522 (US)
(72) Inventor: RAVIKUMAR, Sundaram, Dobbs Ferry, NY 10522 (US); OSBORNE, Guy, Dobbs Ferry, NY 10522 (US); ALWARD, Harry, Allan, Dobbs Ferry, NY 10522 (US); RAVIKUMAR, Vikram, Dobbs Ferry, NY 10522 (US)
(74) Representative: Davies, Gregory Mark
(86) International application number: PCT/US2014/059576
(87) International publication number: WO 2015/054321

(56) References cited:
- WO-A1-2011/025478
- WO-A2-2006/113620
- WO-A2-2007/101983
- WO-A2-2008/139303
- US-A- 5 380 294
- US-A- 5 702 371
- US-A1- 2009 137 961
- US-A1- 2011 060 286
- US-A1- 2013 150 714
- US-B1- 6 443 928

## Description

### FIELD OF THE INVENTION

The present invention relates to medical products, and more particularly to a needle guide assembly through which needles can be easily and accurately placed into a patient's vein.

### BACKGROUND OF THE INVENTION

Intravenous access is a ubiquitous component of some of the most common medical treatments and procedures. Most common access is a percutaneous injection via piercing the skin with a hollow needle inserted into a vein, whether the vein is in the person's hand, arm, groin, neck or other body part. Venipuncture to obtain a blood sample is most commonly obtained from the median cubital vein, which lies within the cubital fossa anterior to the elbow, as this vein lies close to the surface of the skin so it is easily accessible while not surrounded by many nerves so as to minimize the pain for the individual.

Phlebotomy via venipuncture occurs hundreds of millions of time per year worldwide, if not a billion. Accessing a vein is critical, because veins are the conduit through which medical professionals draw blood from as well as inject fluids into a patient's circulatory system.

Most common reasons for venipuncture is to obtain blood for diagnostic purposes, to monitor levels of blood components, and to administer therapeutic treatments such as medications (e.g., intravenous antibiotics), nutrition, or chemotherapy for cancer patients. Other reasons for venipuncture include removing blood due to excess levels of iron (e.g., chelation therapy) or erythrocytes (i.e., red blood cells) or to collect blood for later uses such as donor blood and transfusions. All of these processes require access to a vein via venipuncture.

During conventional venipuncture the user (e.g., phlebotomist, nurse, doctor or other medical personnel) positions the needle to be inserted over the chosen vein at an angle so as to make sure the needle does not puncture the vein and exit the vein on the other side, thus not being in contact with the blood supply within the vein. The user's goal is to position the needle tip within the vein so that there is access to the blood within the vein, whether for blood collection or for insertion of medication, fluids or other compositions into the blood supply of the individual or patient. However, this goal may be complicated as the vein chosen for the venipuncture is typically supported by fatty tissue in the cells that make up the connective tissue, and the chosen vein may lie between the skin and the underlying muscle or bone. So typically the user relies upon visual cues to locate the vein and perform the venipuncture.

Further, the user may position a tourniquet at a location such that the blood increases below the tourniquet and the vein "throbs" making it easier to locate and puncture. The user may also touch and feel the skin of the individual or patient and attempt to locate the vein in this manner, together with a visual observation.

Once the needle tip is inserted into the skin the user may also use tactile senses by trying to "feel" where the needle tip is in relation to the vein. Since the needle tip is inserted into the skin layer, it passes through the fatty tissue layer and into the wall of the vein which acts as a third layer. Depending on the resistance felt by the user, the needle angle may be adjusted as well in order to puncture the vein wall without passing through to the other side of the vein and ensuring contact with the blood within the vein. Once the needle tip is successfully within the vein and in contact with the blood the needle angle may be adjusted depending on the connection at the other end of the needle such as a vacuum bottle for blood collection or an intravenous liquid for insertion into the blood flow of the individual or patient.

In certain instances the individual patient may experience anxiety or even pain depending on the needle tip diameter, the structure of the individual's veins and whether his or her veins have a tendency to collapse when punctured, and the experience level of the user performing the venipuncture. If the venipuncture is not successful there may be repeated attempts until successful completion. Multiple attempts may add to the pain and the anxiety of the individual or patient, especially for a pediatric patient, elderly patient, a special needs individual, or a person with anxiety issues.

One problem which may occur during the venipuncture is movement of the vein targeted for puncture. As the vein is often difficult to hold steady the vein itself being targeted for puncture may roll or move sideways. Further, the needle tip itself on insertion into the skin layer may in fact push the vein to the side and out of the path of the needle tip itself. The patient may also move the body part where the venipuncture is being performed. Upon any of these problems the venipuncture is not successful and further attempts must be made resulting in certain trauma to the individual or patient's skin layer and layers below, as well as to the individual's psyche and anxiety.

In certain circumstances the user may try to use his or her own finger applying pressure to the patient's body part so as to secure the vein from movement, but then the user is exposed for any subsequent sudden movement by the patient and possible puncture of the needle into the user's finger rather than puncturing the vein.

Vein access is critically important especially in patients who need continual access to their circulatory system, such as patients receiving chemotherapy or dialysis. Renal failure is a prevalent chronic disease in the United States with approximately 800,000 patients. The term renal failure refers to the inability of the patient's kidneys to properly rid the patient's blood supply of waste products such as creatinine, urea, and free water. The most common treatment for this condition is hemodialysis. Hemodialysis (or commonly referred to as dialysis) is the process of extra-corporally removing the waste product from the blood supply ;by circulating the patient's blood through a dialysis machine, which "purifies" the Wood of unwanted waste materials and returns the blood to the patient.

There are various techniques in which dialysis is done, but the more desirable and common technique is through what is known as an AV fistula. For the purposes of dialysis, an AV (or Arterial Venous) fistula is a surgical procedure to connect the vein and artery. The AV fistula is accessed by inserting two needles, one needle for drawing the blood to circulate through the dialysis machine and a second needle to return to blood back into the patient.

One common complication with this procedure occurs when the vein is not fully dilated - the needle often does not puncture the vein properly and causes blood to extravasate, causing the swelling and hematoma formation. Worse, improperly puncturing the AV fistula can actually damage the AV fistula making recannulation not possible. Apart from the destruction of the fistula, the patient suffers from pain and discomfort especially from the hematoma, ideally, the needle should enter the vein directly only piercing the vein where it entered. By entering directly into the vein and not puncturing the side or back walls of the vein, the needle can access the AV fistula and allow the dialysis to commence with minimal extravasation and the morbidities associated with it.

Thus, there exists a need for a guide to assist in the proper insertion of a needle into a vein. While certain vein guides have been the subject of patents and applications, the have notable problems. For instance, they may be too binding and constricting on the patient, especially an elderly, pediatric or anxious patient. They may also be too large and wieldy to use easily in a clinic or blood collection site such as Quest Diagnostics, inc. or Laboratory Corporation of America (LabCorp).

A further need exists for a cheaper and easily available guide which may be used by hospitals, clinics and even small individual doctor's offices. A further need exists for a guide which is flexible and not constricting on a patient while in use. A further need exists for a guide which secures the needle once inserted into the individual's body part.

These and other needs are met by the present invention including a needle guide assembly apparatus for vein access and method of use. Other advantages of the present invention will become apparent from the following description and appended claims.

Examples of known apparatus for use in a venipuncture procedure are shown in US6442928 and WO2006/113620.

### BRIEF DESCRIPTION OF THE DRAWINGS

Figure 1 is one embodiment of a needle guide assembly for vein access of the present invention.
Figure 2 is one embodiment of a guide slide assembly of the present invention.
Figure 3 is an exploded view of one embodiment of the present invention.
Figure 4 is a further embodiment of the present invention.
Figure 5 is an embodiment of the present invention and a needle ready for use with the present invention.
Figure 6 is an embodiment of the present invention in use on an arm of a patient and showing a needle in use with the present invention.

### SUMMARY OF THE INVENTION

This invention provides an apparatus for guiding a needle into a vein. The invention further provides an apparatus for securing an inserted needle into a vein during venipuncture.

The invention includes a needle guide assembly for vein access, as defined in appended claim 1. The vein access needle guide assembly is the conduit through which the needle is guided, inserted and may be secured during venipuncture. A portion of the vein access needle guide assembly is transparent or translucent to allow the user performing the venipuncture to properly align the needle with the target vein in an individual or patient.

In one embodiment, the vein access needle guide assembly apparatus includes a retaining means comprising an adhesive backed plastic piece with an aperture such as a hole, functioning as a guide, in the middle of the plastic piece, in this embodiment the aperture may have a "track" that when aligned properly above the targeted vein or even a fistula, would guide the needle into the vein at the proper angle, therefore reducing the risk of extravasation. The vein access needle guide assembly may include additional portions joined on each side of a guide slide assembly to secure the vein access needle guide assembly to the patient's skin when in use, which have an adhesive backing or other configuration to be affixed onto the skin.

The method of use of the invention (which is not part of the claimed invention) includes placing the inventive vein access needle guide assembly onto the body part of the individual or patient at the location of the target vein or fistula, retaining the vein access needle guide assembly on the skin such as by adhesion, inserting the needle into the groove and aperture of the vein access needle guide assembly, puncturing the vein while at the same time securing the needle in place within the vein while extending out of the skin.

Other embodiments of the inventive apparatus and method are disclosed below.

### DETAILED DESCRIPTION OF THE INVENTION

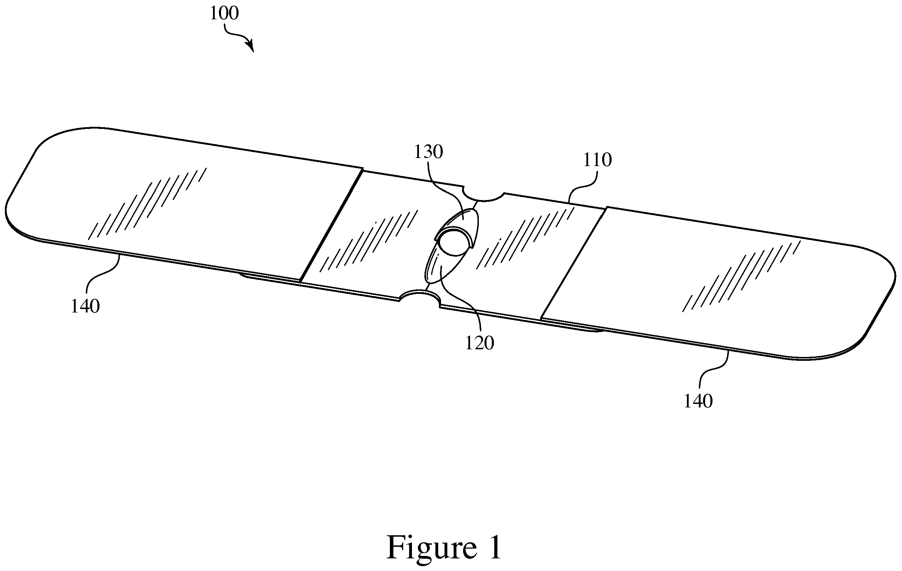

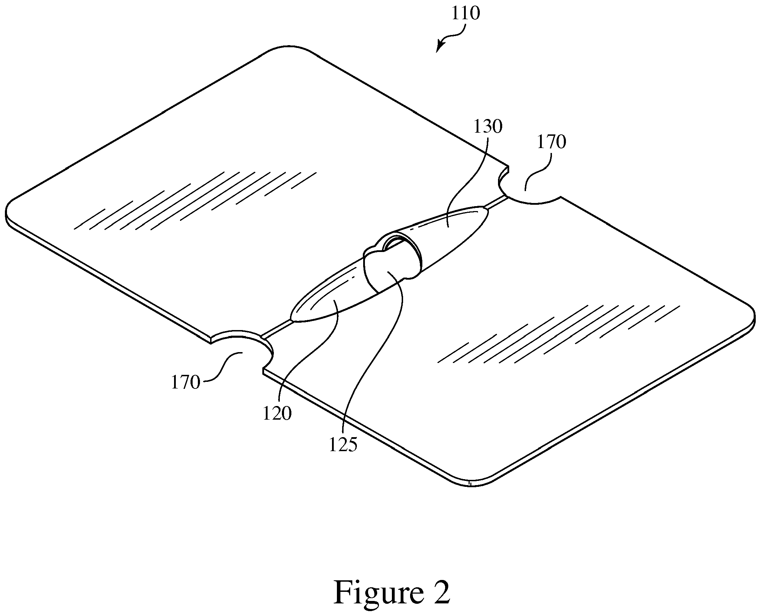

Reference will now be made to the drawings wherein like reference numerals may identify similar structural features or aspects of the subject invention. For purposes of explanation and illustration, and not limitation, a view of an exemplary embodiment of the needle guide assembly for vein access is shown in Figure 1 and designated generally by reference character 100. Other embodiments of the vein access needle guide assembly and methods in accordance with the invention, or aspects thereof, are provided in Figures 2 through 6.

Referring now to Figures 1 through 6, a vein access needle guide assembly ***100*** is shown to have a guide slide assembly ***110*** including a guide slide comprising a concave portion ***120*** and a convex portion ***130***. The guide slide assembly ***110*** may be made out of a rigid or semi-rigid material and could be translucent or transparent. The guide slide assembly ***110*** has convex ***130*** and concave ***120*** portions to accommodate and guide a needle as it is inserted into the patient. Further, the convex portion ***130*** of the guide slide assembly ***110*** may secure the needle as it will overlay the needle when inserted into the patient's skin during the venipuncture. In certain embodiments the convex and concave portions may be reversed, or only one portion may be included, either the convex portion alone or the concave portion alone.

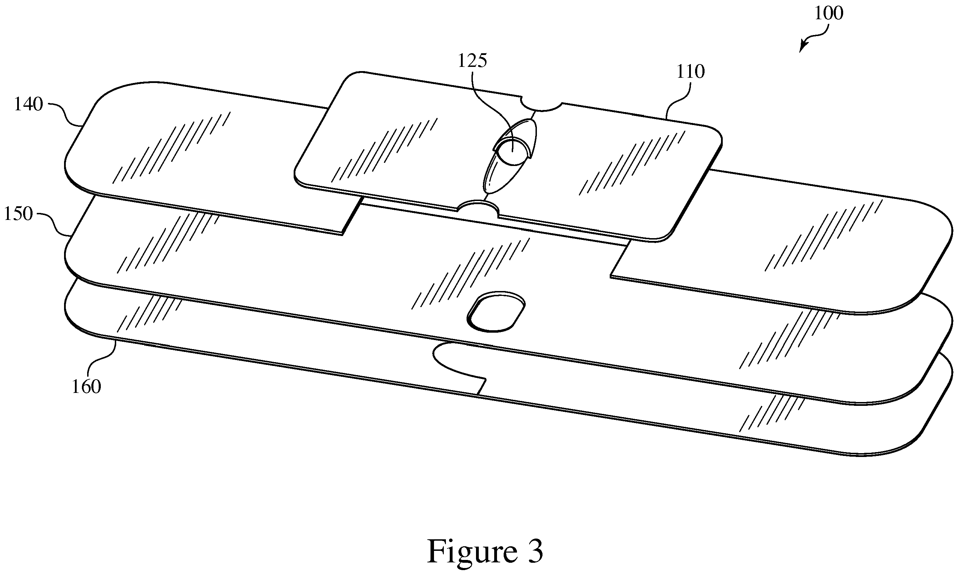

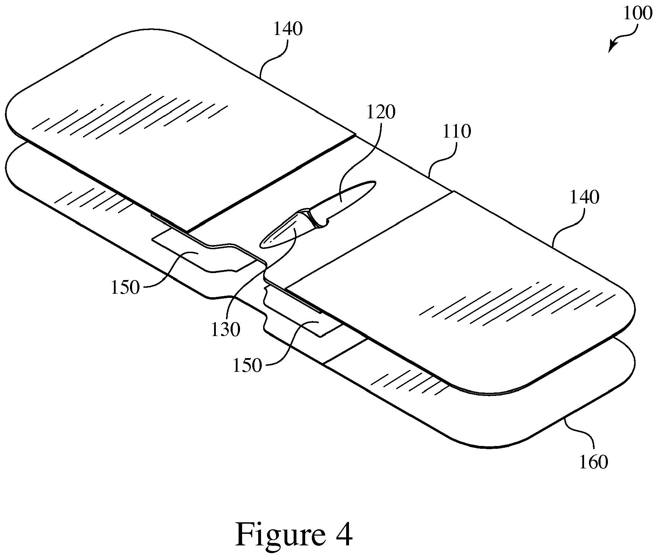

The vein access needle guide assembly ***100*** further includes a retaining means such as at least one adhesive side strap ***140*** which secures the vein access needle guide assembly ***100*** to the skin of the patient when in use. The adhesive side strap ***140*** may be comprised of a polymer film, plastic, nonwoven or woven material, or the material may be combinations thereof. This material is preferably non-absorbent or essentially non-absorbent and preferably flexible. The adhesive of the adhesive side strap ***140*** should be capable of maintaining the adhesive property when wet given the possibility of blood or liquid medications being in contact with the vein access needle guide assembly ***100*** when in use. The adhesive may be contained on the adhesive side strap ***140*** itself or the adhesive may be applied to both the adhesive side strap ***140*** and guide slide assembly ***110*** in the same step when producing the vein access needle guide assembly ***100***. Such difference may be seen in Figures 3 and 4 wherein the adhesive layer ***150*** is either a longer strip layer below the adhesive side strap ***140*** and guide slide assembly ***110*** while above the paper layer ***160***, as shown in Figure 3. In another embodiment the adhesive is broken into portions and located only below the guide slide assembly ***110***, as the adhesive side strap ***140*** is provided with a separate adhesive layer prior to assembly of the vein access needle guide assembly ***100***. In certain embodiments the adhesive layer ***150*** may have an opening or aperture which corresponds with the aperture ***125*** of the guide slide assembly ***110***, or may be smaller or may be larger than the aperture ***125**,* or even have a multiple of openings, in one embodiment the opening or aperture in the adhesive layer ***150*** is of a size such that the convex portion opening is unencumbered (i.e., is afforded clear access) through the adhesive layer ***150*** so as to permit entry of the needle tip for reaching the skin., In yet another embodiment the adhesive layer ***150*** may be a solid layer below the aperture ***125***. In an alternate embodiment the adhesive layer ***150*** may be as shown in Figure 4.

The adhesive side strap ***140*** includes a layer of adhesive on the bottom to adhere to the patient's skin when in use. The adhesive may be acrylic, polymeric or any natural or synthetic adhesive capable of adhering to a patient's skin when in use and either re-adhering or capable of being removed from the patient's skin with some force but not enough to remove the skin layer of the patient. Any combinations of the adhesives named and those known in the art may be used. IN general any retaining means may be employed which is capable of retaining the needle guide assembly to the skin of the patient while in use.

The vein access needle guide assembly ***100*** may be produced in a plurality of sizes, with different sized guide slide assemblies ***110*** to accommodate the different standard needle gauges used by medical professionals. One example is a rectangular vein access needle guide assembly with dimensions of about 5 mm to about 50 mm wide and about 10 mm to about 100 mm long with the concave portion ***120*** and convex portion ***130*** forming an aperture ***125*** with a diameter of about 0.5 mm to about 10 mm. These dimensions may be changed depending on the patient's size and the size of the needle. For example, the size may be changed dependent on the target patient, such as children compared to adults. In one example the dimensions are about 10 mm to about 30 mm wide and about 45 mm to about 75 mm long with the concave portion ***120*** and convex portion ***130*** forming an aperture ***125*** with a diameter of about 1.0 mm to about 3.5 mm.

The angle of the guide slide ***110*** is to be configured to appropriately guide the needle into the vein without causing extravasation. Those appropriately trained in the art will appreciate that if a needle is inserted at the wrong angle or too far within a vein that it will damage the vein wall, causing discomfort, bruising, and even hematoma.

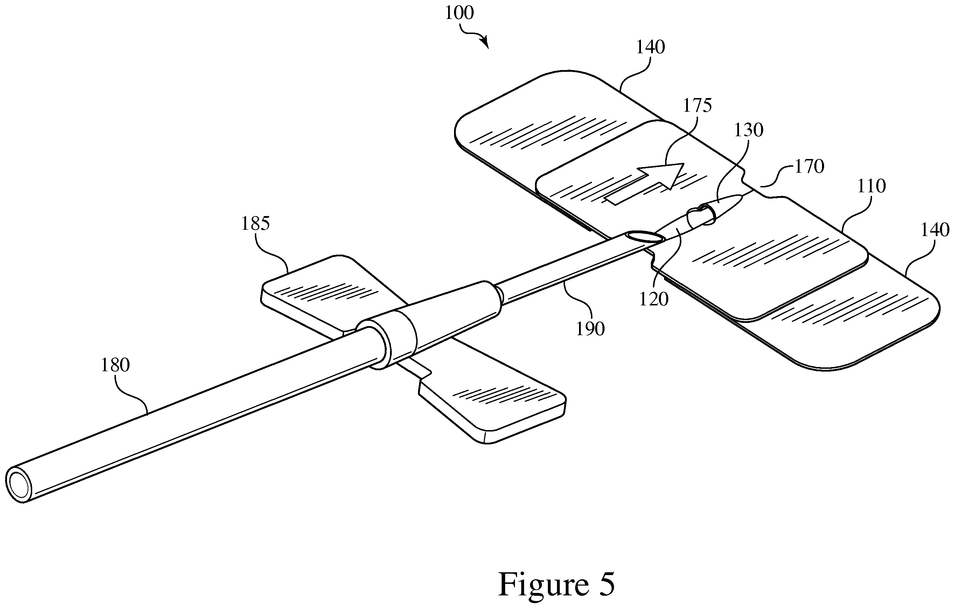

Figure 2 shows the guide slide assembly ***110*** in more detail. The guide slide assembly ***110*** has a concave portion ***120*** and convex portion ***130*** forming an aperture ***125***. The aperture ***125*** may be defined only by the edges of each of the concave portion ***120*** and convex portion ***130*** or it may be larger when viewed from a top view. The guide slide assembly ***110*** may optionally include one or more guide notches ***170**.* In a further embodiment shown in Figure 5 the guide slide assembly ***110*** may include a directional indicator ***175**,* which may be a graphic, such as an arrow pointing in the direction for the user to insert the needle into the vein access guide slide assembly ***100***. The direction indicator ***175*** could be comprised of words such as "INSERT HERE" or "UP" or any combination of graphics and/or words.

In an alternate embodiment of the guide slide assembly ***110*** only a concave portion ***120*** is included to guide the needle into the patient's skin when in use. In this embodiment the remaining portion of the guide slide assembly ***110*** is flat and flush with the surface of the patient's skin. Again, an optional graphic may be included. In yet another embodiment of the guide slide assembly ***110*** has a concave portion ***120*** which is formed to extend beneath the underside of the guide slide assembly ***110***. In a further embodiment the concave portion ***130*** may be formed as a depression in the guide slide assembly ***110***, Other combinations are possible.

Reference character ***110*** of Figures 3 and 4 is a vein access needle guide assembly with adhesive-backed side straps ***140*** shown; such an assembly would further allow the vein access needle guide assembly ***100*** to affix securely atop the patient's skin without slipping off. Figure 3 shows one embodiment of the vein access needle guide assembly ***100*** with the guide slide assembly ***110*** in a position on top of the adhesive-backed side straps ***140*** while Figure 4 is a different embodiment of the vein access needle guide assembly ***100*** wherein the guide slide assembly ***110*** is in a position with the edges of one distal end affixed below a distal end of the adhesive-backed side straps ***140***.

Figure 3 is an exploded view of one embodiment of the vein access needle guide assembly ***100*** with the different parts shown. The vein accessible needle guide assembly ***100*** with two side straps ***140*** to be affixed on each side of a distal end of the guide slide assembly ***110*** is shown with an adhesive layer ***150*** as well as a paper liner layer ***160*** that protects the adhesive portion until use. It may be preferable that the paper liner layer ***160*** have off-set peel tabs (not shown).

Figure 5 shows a needle ***190*** entering into the vein access needle guide with side straps ***110*** that are affixed atop of a patient's skin. It can be noted that the needle slide ***110*** is aligned atop of the vein so that when a needle is inserted it will enter the cannula of the vein.

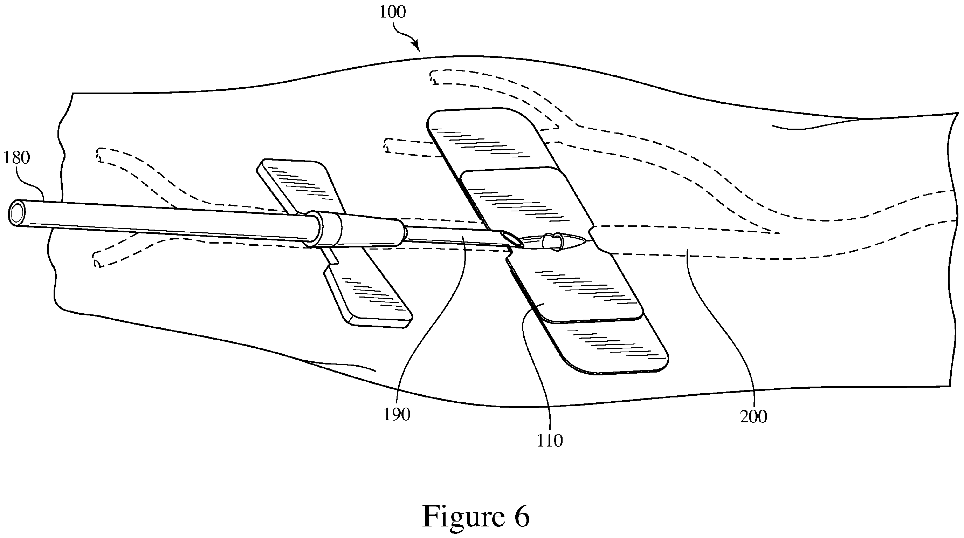

A needle ***180*** is also shown in Figure 5 where the needle tip ***190*** is on the edge of the concave portion ***120*** of the guide slide assembly ***110*** but not within the aperture ***125***. The needle ***180*** may be any conventional needle having a needle tip ***190*** of various diameters capable of insertion into the aperture ***125***. The needle shown in Figures 5 and 6 is one example of a needle ***180*** and in this embodiment is a winged-tip butterfly needle including a winged stabilizer ***185***. Some examples of needle tip ***190*** diameters are about 1.8 mm to about 2.2 mm These dimensions may change depending on the size of the needle. In use, the needle ***180*** is moved in a direction to correspond to the flow of blood in the target vein, and in this instance is moved such that the needle tip ***190*** is aligned with the concave portion ***120*** of the guide slide assembly ***110*** and moves into the aperture ***125*** and is inserted into the patient's skin, with the convex portion ***130*** and the entire guide slide assembly ***110*** and the vein access needle guide assembly ***100*** securing the needle ***180*** while in use.

It is envisioned that the vein access needle guide assembly ***100*** would be packaged in a sterilized package, either as a stand-alone product or in combination with multiple needle guides or other products such as a needle. Other kits including the vein access needle guide assembly ***100*** could include a cleansing agent in a sterile package, a tourniquet, the vein access needle guide assembly ***100,*** a needle, a piece of sterile gauze and a sterile bandage.

A method of venipuncture using the inventive vein access guide assembly ***100*** may include a number of steps. First, the target vein is chosen (if an AV fistula is not the target location) such as on the outside of the forearm, on the back of the hand, in the antecubital fossa or any other body location. Next, the skin on the patient located where the user wants to access the vein is cleaned by a cleansing agent. The cleansing agent used to prepare the insertion site may be iodine, povidone-iodine, or ethyl alcohol. The cleansing agent may be an alcohol swab, preferably 70% isopropyl alcohol. The vein is palpated, and to dilate the vein a tourniquet may be wrapped around the arm proximal to the intended site of puncture Optionally a tourniquet may be placed at a location near the target vein so that the target vein protrudes or is more easily visualized or subject to tactile manipulation and touch. The patient may be asked to pump his or her fist if the target vein is located in the arm.

Next, if the vein access needle guide ***100*** is packaged in a sterile packaging, then packaging is opened. The vein access guide assembly ***100*** is aligned with the target vein such that the concave portion ***120*** is aligned with the flow of the blood in the target vein as the inserted needle would also align with the blood flow. The paper liner layer ***160*** is peeled back exposing the adhesive layer ***150*** and the vein access needle guide ***100*** is affixed atop the patient's skin, appropriately aligning over the target vein, preferably with the needle guide slide ***120*** located over the target vein. As shown in Figure 6, a needle ***180*** is inserted into the guide slide ***110*** to access the vein and the concave portion ***120*** secures the needle ***180***. The angle of the needle tip ***190*** may be between about 5° to about 90°, preferably about 10° to about 40°, and more preferably about 15° to about 30°, dependent on the diameter of the needle tip and the size of the target vein ***200***. After the skin is punctured, little resistance should be felt by the user as the needle tip ***190*** should pass through the subcutaneous tissue, but a sudden slight resistance may be felt as the needle tip ***190*** hits the wall of the vein. At this point the needle tip ***190*** may be cautiously advanced, with the needle ***180*** held nearly flush with the skin as the needle ***180*** is in the concave portion ***120*** of the guide slide assembly ***110*** and secured by the convex portion ***130***. Slight upward pressure applied by the user and the guide slide assembly ***110*** itself may aid in keeping the needle tip ***190*** in the target vein ***200*** as the needle tip ***190*** is advanced into the lumen of the target vein ***200***. A successful venipuncture results in blood flowing back into the hub of the needle ***180*** or into the catheter or vacuum tube (neither shown) attached to the distal end of the needle ***180***, and the needle tip ***190*** usually can be felt to be in the vein. If these signs are absent, the needle tip ***190*** is not in the target vein ***200**,* in which case it is usually best to remove the needle ***180***, apply pressure to the puncture site, and start the procedure again, using new equipment.

In a successful venipuncture using the inventive vein access needle guide assembly ***100***, the user now has access to the patient's vein and blood may be drawn and fluids or medication put into the circulatory system. At a later time the needle ***180*** is removed from the patient's vein by the user, the vein access needle guide assembly ***100*** is removed, and optionally a sterile piece of gauze, and a conventional adhesive bandage is placed atop the location on the patient's skin where the vein was accessed to provide pressure until the local bleeding stops.

The inventive method may be used to obtain blood for diagnostic purposes, to monitor levels of blood components, to administer therapeutic treatments such as medications (*e.g*., intravenous antibiotics), nutrition, or chemotherapy for cancer patients. Other uses of the inventive venipuncture method and other uses for inventive vein access needle guide assembly ***100*** include removing blood due to excess levels of iron (*e.g*., chelation therapy) or erythrocytes (*i.e*., red blood cells) or to collect blood for later uses such as donor blood and transfusions. Other uses may be contemplated if relating to insertion of a needle into a patient's skin to access a vein.

The inventive vein access needle guide assembly ***100*** assists the user in the proper insertion of a needle into a vein. The inventive vein access needle guide assembly ***100*** is portable and does not impinge on the patient's skin but rather is easily placed on the patient's skin in the location of the target vein and is retained on the skin sufficiently for the venipuncture and securing of the needle without being so secure that the user cannot remove the vein access needle guide assembly ***100*** easily with minimal force. The vein access needle guide assembly ***100*** of the present invention may lessen anxiety and pain for the patient in that that vein access needle guide assembly ***100*** is small and pliable while also assisting in the venipuncture which results in a quicker venipuncture process, as well as a more accurate process. Further, the user may experience less anxiety also based on the needle guide slide ***110*** of the inventive vein access needle guide assembly ***100*** and being able to easily access the target vein and not having to rely on the user's own visual cues or tactile impressions such that the risk of puncture of the user's finger is lessened or even totally removed.

Further, the inventive method and inventive vein access needle guide assembly ***100*** may be used in venipuncture related to a fistula, including an AV fistula. The patient may therefore experience less pain and the user experience a quicker and/or easier and/or more accurate and less stressful, venipuncture of the skin surrounding the AV fistula.

The inventive method and inventive vein access needle guide assembly ***100*** may be cheaper and more easily available than current vein guide apparatuses and thus may be used by hospitals, clinics and even small individual doctor's offices.

The invention has been described in terms of embodiments thereof, but is more broadly applicable as will be understood by those skilled in the art. The scope of the invention is only limited by the following claims.

## Claims

1. A needle guide assembly (100) for vein access, comprising
a guide having a peripheral edge, a convex portion (130), a concave portion (120) and an aperture (125), the concave portion (120) and the convex portion (130) meeting each other to define the aperture (125) between the concave portion (120) and the convex portion (130), each of the convex portion (130) and the concave portion (120) extending away from the aperture (125) in opposite directions from each other in a tapering manner along a longitudinal axis of the guide and terminating the tapering at respective locations away from the peripheral edge, the convex portion (130) being made of a transparent or translucent material; and
retaining means (140) for retaining the guide to skin.

2. The assembly of claim 1, further **characterized by** a kit that contains a sterile package enclosing the guide, the means (140) for retaining the guide to skin, a packaged sterile alcohol swab, a packaged sterile needle and a packaged sterile bandage.

3. The assembly of claim 1 or 2, **characterized in that** the convex portion (130) extends from a topside of the guide, the concave portion (120) extends from an underside of the guide.

4. The assembly of claim 1 or 2, **characterized in that** the retaining means includes an adhesive layer having an opening in alignment with at least one of the concave (120) and convex (130) portions.

5. The assembly of claim 4, wherein the concave portion (120) fits into the opening in the adhesive layer (150).

6. The assembly of claim 1 or 2, **characterized in that** the guide has two sets of pairs of opposite edges that form the periphery, the convex portion (130) and the concave portion (120) each being symmetric relative to a line of symmetry, further comprising two side straps (140) adhered to the guide and extending outward past one of the sets of pairs of the opposite edges, each of the two side straps (140) having a respective edge spaced away from each other that are substantially equidistant from the line of symmetry.

7. The assembly of claim 1 or 2, **characterized in that**:
i) the guide has two sets of pairs of opposite edges that form the periphery, the opposite edges of one of pairs of the two sets each defining a respective notch (170) so as to provide for two notches (170), the concave portion (120) and the convex portion (130) extending from the aperture (125) away from each other between the two notches (170) so that the convex portion (130) tapers in a direction toward one of the two notches (170) and the concave portion (120)tapers in a direction toward a remaining one of the two notches (170); and/or
ii) the guide has a direction indicator (175) marked on a topside of the guide that points in a same direction that the convex portion (130) tapers.

8. The assembly of claim 1 or claim 2, wherein the guide has two sets of pairs of opposite edges that form the periphery, at least one of the opposite edges of one of the pairs of the two sets defining a notch (170), at least one of the convex portion (130) and the concave portion (120) tapering in a direction toward the notch (170).

9. The assembly of claim 8, wherein said retaining means (140) includes an adhesive layer (150) that has a further notch in alignment with the notch (170) of the guide and being substantially identical in shape to that of the notch of the guide.

10. The assembly of claim 1, **characterized by** a needle stabilizer (185) having a channel that extends in a channel direction to accommodate insertion of a needle (180), spacing the needle stabilizer (185) from the guide by a distance sufficient to enable simultaneous insertion of the needle (180) through both the channel of the needle stabilizer (185) and the aperture (125) of the guide in alignment so that a tip (190) of the needle (180) enters the convex portion (130) of the guide as a portion of a remainder of the needle (180) is within the channel.

11. The assembly of claim 1 or 2, **characterized in that**:
i) the retaining means (140) includes an adhesive layer (150), the adhesive layer (150) having an opening accommodating insertion of the concave portion (120) within the opening and defining a space in alignment with the convex portion (130); and/or
ii) the retaining means (140) includes an adhesive layer (150), that adhesive layer (150) having an adhering side, further **characterized by** a paper layer (160) against the adhering side of the adhesive layer (150) and peelable under manual force for removal from the adhering side; and/or
iii) the convex portion (130) extends from a topside of the guide, the topside of the guide having a depression forming the concave portion (120).

## Patentansprüche

1. Nadelführungsanordnung (100) für Venenzugang, umfassend
eine Führung mit einem Umfangsrand, einem konvexen Abschnitt (130), einem konkaven Abschnitt (120) und einer Öffnung (125), wobei sich der konkave Abschnitt (120) und der konvexe Abschnitt (130) treffen, um die Öffnung (125) zwischen dem konkaven Abschnitt (120) und dem konvexen Abschnitt (130) zu definieren, wobei der konvexe Abschnitt (130) und der konkave Abschnitt (120) jeweils weg von der Öffnung (125) in entgegengesetzte Richtungen voneinander, sich verjüngend entlang einer Längsachse der Führung verlaufen und die Verjüngung an jeweiligen Stellen entfernt von dem Umfangsrand endet, wobei der konvexe Abschnitt (130) aus einem transparenten oder lichtdurchlässigen Material hergestellt ist; und
Haltemittel (140) zum Halten der Führung an der Haut.

2. Anordnung nach Anspruch 1, ferner **gekennzeichnet durch** ein Kit, das eine sterile Packung, die die Führung umschließt, das Mittel (140) zum Halten der Führung an der Haut, einen verpackten sterilen Alkoholtupfer, eine verpackte sterile Nadel und einen verpackten sterilen Verband enthält.

3. Anordnung nach Anspruch 1 oder 2, **dadurch gekennzeichnet, dass** der konvexe Abschnitt (130) von einer Oberseite der Führung ausgeht und der konkave Abschnitt (120) von einer Unterseite der Führung ausgeht.

4. Anordnung nach Anspruch 1 oder 2, **dadurch gekennzeichnet, dass** das Haltemittel eine Klebstoffschicht mit einer Öffnung, die mit mindestens einem von dem konkaven (120) und dem konvexen (130) Abschnitt ausgerichtet ist, umfasst.

5. Anordnung nach Anspruch 4, wobei der konkave Abschnitt (120) in die Öffnung in der Klebstoffschicht (150) passt.

6. Anordnung nach Anspruch 1 oder 2, **dadurch gekennzeichnet, dass** die Führung zwei Sätze von Paaren gegenüberliegender Ränder aufweist, die den Umfang bilden, wobei der konvexe Abschnitt (130) und der konkave Abschnitt (120) jeweils symmetrisch um eine Symmetrielinie sind, ferner umfassend zwei Seitenlaschen (140), die an der Führung angebracht sind und an einem der Sätze von Paaren der gegenüberliegenden Ränder vorbei nach außen verlaufen, wobei jede der zwei Seitenlaschen (140) einen jeweiligen Rand aufweist, die beide voneinander beabstandet sind und im Wesentlichen abstandsgleich von der Symmetrielinie sind.

7. Anordnung nach Anspruch 1 oder 2, **dadurch gekennzeichnet, dass:**
i) die Führung zwei Sätze von Paaren gegenüberliegender Ränder aufweist, die den Umfang bilden, wobei die gegenüberliegenden Ränder eines Paares der zwei Sätze jeweils eine jeweilige Kerbe (170) definieren, so dass zwei Kerben (170) bereitgestellt werden, wobei der konkave Abschnitt (120) und der konvexe Abschnitt (130) von der Öffnung (125) weg voneinander zwischen den zwei Kerben (170) verlaufen, so dass der konvexe Abschnitt (130) sich in Richtung zu einer der zwei Kerben (170) verjüngt und der konkave Abschnitt (120) sich in einer Richtung zu einer verbleibenden der zwei Kerben (170) verjüngt; und/oder
ii) die Führung eine Richtungsanzeige (175) aufweist, die auf einer Oberseite der Führung angegeben ist und die in die gleiche Richtung weist, in der sich der konvexe Abschnitt (130) verjüngt.

8. Anordnung nach Anspruch 1 oder Anspruch 2, wobei die Führung zwei Sätze von Paaren gegenüberliegender Ränder aufweist, die den Umfang bilden, wobei mindestens einer der gegenüberliegenden Ränder eines der Paare der zwei Sätze eine Kerbe (170) bildet, wobei sich mindestens einer des konvexen Abschnitts (130) und des konkaven Abschnitts (120) in einer Richtung zur Kerbe (170) verjüngt.

9. Anordnung nach Anspruch 8, wobei das Haltemittel (140) eine Klebstoffschicht (150) aufweist, die eine weitere Kerbe aufweist, die mit der Kerbe (170) der Führung ausgerichtet ist und in der Form im Wesentlichen identisch mit der Kerbe der Führung ist.

10. Anordnung nach Anspruch 1, **gekennzeichnet durch** einen Nadelstabilisator (185) mit einem Kanal, der in einer Kanalrichtung verläuft, um die Einführung einer Nadel (180) aufzunehmen, wobei der Nadelstabilisator (185) von der Führung um einen ausreichenden Abstand entfernt ist, um gleichzeitige Einführung der Nadel (180) durch sowohl den Kanal des Nadelstabilisators (185) als auch die Öffnung (125) der damit ausgerichteten Führung zu ermöglichen, so dass eine Spitze (190) der Nadel (180) in den konvexen Abschnitt (130) der Führung eintritt, während ein Abschnitt eines Rests der Nadel (180) innerhalb des Kanals ist.

11. Anordnung nach Anspruch 1 oder 2, **dadurch gekennzeichnet, dass:**
i) das Haltemittel (140) eine Klebstoffschicht (150) einschließt, wobei die Klebstoffschicht (150) eine Öffnung aufweist, die die Einführung des konkaven Abschnitts (120) innerhalb der Öffnung aufnimmt und einen Raum definiert, der mit dem konvexen Abschnitt (130) ausgerichtet ist; und/oder
ii) das Haltemittel (140) eine Klebstoffschicht (150) einschließt, wobei die Klebstoffschicht (150) eine Haftseite aufweist, ferner **gekennzeichnet durch** eine Papierschicht (160) an der Haftseite der Klebstoffschicht (150), die zum Entfernen von der Haftseite mit manueller Kraft abziehbar ist; und/oder
iii) der konvexe Abschnitt (130) von einer Oberseite der Führung ausgeht, wobei die Oberseite der Führung eine Vertiefung aufweist, die den konkaven Abschnitt (120) bildet.

## Revendications

1. Ensemble guide-aiguille (100) pour accès veineux, comprenant
un guide ayant un bord périphérique, une partie convexe (130), une partie concave (120) et un orifice (125), la partie concave (120) et la partie convexe (130) se rencontrant pour définir l'orifice (125) entre la partie concave (120) et la partie convexe (130), chacune de la partie convexe (130) et de la partie concave (120) s'étendant à partir de l'orifice (125) dans des directions opposées en s'effilant dans un axe longitudinal du guide et finissant leur effilement à des endroits respectifs éloignés du bord périphérique, la partie convexe (130) étant faite d'un matériau transparent ou translucide ; et
des moyens de retenue (140) pour retenir le guide contre la peau.

2. Ensemble selon la revendication 1, **caractérisé en outre par** une trousse qui contient un emballage stérile enfermant le guide, les moyens (140) pour retenir le guide contre la peau, une compresse imbibée d'alcool sous emballage stérile, une aiguille sous emballage stérile et une bande sous emballage stérile.

3. Ensemble selon la revendication 1 ou 2, **caractérisé en ce que** la partie convexe (130) s'étend à partir d'une face supérieure du guide, la partie concave (120) s'étend à partir d'une face inférieure du guide.

4. Ensemble selon la revendication 1 ou 2, **caractérisé en ce que** les moyens de retenue comprennent une couche adhésive ayant une ouverture dans l'alignement d'au moins une des parties concave (120) et convexe (130).

5. Ensemble selon la revendication 4, dans lequel la partie concave (120) s'ajuste dans l'ouverture de la couche adhésive (150).

6. Ensemble selon la revendication 1 ou 2, **caractérisé en ce que** le guide a deux jeux de paires de bords opposés qui forment la périphérie, la partie convexe (130) et la partie concave (120) étant chacune symétrique par rapport à une ligne de symétrie, comprenant en outre deux languettes latérales (140) collées au guide et s'étendant vers l'extérieur au-delà de l'un des jeux de paires des bords opposés, chacune des deux languettes latérales (140) ayant des bords respectifs éloignés l'un de l'autre qui sont essentiellement équidistants de la ligne de symétrie.

7. Ensemble selon la revendication 1 ou 2, **caractérisé en ce que** :
i) le guide a deux jeux de paires de bords opposés qui forment la périphérie, les bords opposés de l'une des paires des deux jeux définissant respectivement une encoche (170) de façon à former deux encoches (170), la partie concave (120) et la partie convexe (130) s'étendant à partir de l'orifice (125) en s'éloignant l'une de l'autre entre les deux encoches (170) de sorte que la partie convexe (130) s'effile en direction de l'une des deux encoches (170) et la partie concave (120) s'effile en direction de l'autre des deux encoches (170) ; et/ou
ii) le guide a un indicateur de direction (175) marqué sur une face supérieure du guide, qui pointe dans la même direction que celle dans laquelle la partie convexe (130) s'effile.

8. Ensemble selon la revendication 1 ou 2, dans lequel le guide a deux jeux de paires de bords opposés qui forment la périphérie, au moins un des bords opposés de l'une des paires des deux jeux définissant une encoche (170), au moins une de la partie convexe (130) et de la partie concave (120) s'effilant en direction de l'encoche (170).

9. Ensemble selon la revendication 8, dans lequel lesdits moyens de retenue (140) comprennent une couche adhésive (150) qui a une encoche supplémentaire dans l'alignement de l'encoche (170) du guide et dont la forme est essentiellement identique à celle de l'encoche du guide.

10. Ensemble selon la revendication 1, **caractérisé par** un stabilisateur d'aiguille (185) ayant un conduit qui s'étend dans une direction de conduit pour accueillir l'insertion d'une aiguille (180), éloignant le stabilisateur d'aiguille (185) du guide d'une distance suffisante pour permettre l'insertion simultanée de l'aiguille (180) dans le conduit du stabilisateur d'aiguille (185) et dans l'orifice (125) du guide situé dans l'alignement, de sorte qu'une pointe (190) de l'aiguille (180) entre dans la partie convexe (130) du guide alors qu'une partie du reste de l'aiguille (180) se trouve à l'intérieur du conduit.

11. Ensemble selon la revendication 1 ou 2, **caractérisé en ce que :**
i) les moyens de retenue (140) comprennent une couche adhésive (150), la couche adhésive (150) ayant une ouverture accueillant l'insertion de la partie concave (120) dans l'ouverture et définissant un espace dans l'alignement de la partie convexe (130) ; et/ou
ii) les moyens de retenue (140) comprennent une couche adhésive (150), ladite couche adhésive (150) ayant une face adhésive, **caractérisé en outre par** une couche de papier (160) contre la face adhésive de la couche adhésive (150) et pelable par une force manuelle pour être retirée de la face adhésive ; et/ou
iii) la partie convexe (130) s'étend à partir d'une face supérieure du guide, la face supérieure du guide ayant une dépression formant la partie concave (120).
